# EUROPÄISCHE PATENTANMELDUNG

(11) **EP 4 147 787 A1**
(43) Veröffentlichungstag der Anmeldung: **15.03.2023**
(21) Anmeldenummer: 21196124.8
(22) Anmeldetag: 10.09.2021
(51) Int. Cl.: B05B 11/00, A61F 9/00

(54) **FLÜSSIGKEITSSPENDER**

(71) Anmelder: Aptar Radolfzell GmbH, 78315 Radolfzell (DE)
(72) Erfinder: Greiner-Perth, Jürgen, 78244 Gottmadingen (DE)
(74) Vertreter: Witte, Weller und Partner Patentanwälte mbB Stuttgart

(57) **Zusammenfassung**

Vorgeschlagen wird ein Tropfenspender (10) zum tropfenweisen Austrag einer Flüssigkeit, insbesondere zum tropfenweisen Austrag einer pharmazeutischen Flüssigkeit. Dieser weist einen Flüssigkeitsspeicher (14) zur Lagerung der Flüssigkeit vor dem Austrag sowie eine Austragöffnung (16) auf, der eine Tropfenbildungsgeometrie (18) zugeordnet ist. Weiterhin weist der Tropfenspender (10) weist eine Pumpeinrichtung (40) auf, mittels derer Flüssigkeit aus dem Flüssigkeitsspeicher (14) zur Austragöffnung (16) gefördert werden kann, so dass die Flüssigkeit sich an der Tropfenbildungsgeometrie (18) sammelt und sich bei ausreichender gesammelter Flüssigkeitsmenge von der Tropfenbildungsgeometrie (18) löst.

Der erfindungsgemäße Tropfenspender (10) weist ein flaches Gehäuse (12) auf, innerhalb dessen der Flüssigkeitsspeicher und die Pumpeinrichtung angeordnet sind, wobei die Außenform des flachen Gehäuses (12) eine maximale Breite orthogonal zu einer Hauptachse (2) des Gehäuses aufweist, die mindestens 25% größer als die maximale Dicke der Außenform des Gehäuses (12) orthogonal zur Hauptachse und orthogonal zur Breite ist. Zur Betätigung der Pumpeinrichtung (40) ist an einer Seitenfläche des Gehäuses (12) eine eindrückbare Betätigungshandhabe (24) vorgesehen.

## Beschreibung

### ANWENDUNGSGEBIET UND STAND DER TECHNIK

Die Erfindung betrifft einen Tropfenspenderzum tropfenweisen Austrag einer Flüssigkeit, insbesondere zum tropfenweisen Austrag einer pharmazeutischen Flüssigkeit. Ein solcher Tropfenspender kann beispielsweise für ophthalmische Zwecke Verwendung finden.

Ein erfindungsgemäßer Tropfenspender weist ebenso wie gattungsgemäße Tropfenspender einen Flüssigkeitsspeicher zur Lagerung der Flüssigkeit vor dem Austrag auf sowie eine Pumpeinrichtung, mittels derer Flüssigkeit aus dem Flüssigkeitsspeicher zu einer Austragöffnung des Tropfenspenders gefördert werden kann. Der Austragöffnung ist eine Tropfenbildungsgeometrie zugeordnet, so dass die Flüssigkeit sich an dieser Tropfenbildungsgeometrie sammeln kann und sich bei ausreichender gesammelter Flüssigkeitsmenge von der Tropfenbildungsgeometrie als Einzeltropfen löst.

Derartige Tropfenspender gestatten einen gut dosierbaren Austrag von Flüssigkeit.

### AUFGABE UND LÖSUNG

Aufgabe der Erfindung ist es, einen Tropfenspender zur Verfügung zu stellen, der im Alltag unproblematisch und sicher mitgeführt werden kann und der in angenehmer Weise verwendbar ist.

Erfindungsgemäß wird zu diesem Zweck ein Tropfenspender gattungsgemäßer Art vorgeschlagen, der über die beschriebenen Komponenten eines Flüssigkeitsspeichers, einer Pumpeinrichtung und einer Austragöffnung verfügt. Im verkaufsfertigen Zustand ist der Flüssigkeitsspeicher vorzugweise mit einer pharmazeutischen Flüssigkeit befüllt. Insbesondere kann es sich um eine konservierungsmittelfreie Flüssigkeit handeln, wobei der Spender zur Nutzung mit einer solchen konservierungsmittelfreien Flüssigkeit derart ausgestaltet ist, dass ein Keimeintrag von außen wirksam verhindert wird, insbesondere durch ein sicher schließendes Austragventil und/oder durch einen Belüftungskanal, der mit einem Sterilfilter versehen ist, durch den mindestens 99,9% der Keime aus eintretender Luft entfernt werden. Der Belüftungskanal kann zusätzlich mit einem Belüftungsventil versehen sein.

Die Austragöffnung ist im Bereich einer Tropfenbildungsgeometrie vorgesehen, die zur Ausbildung von Einzeltropfen von im Wesentlichen reproduzierbarer Größe geeignet ist. Es kann sich beispielsweise um eine kelchförmige Tropfenbildungsfläche oder um eine von einer Abrisskante umgebene und im Wesentlichen plane Tropfenbildungsfläche handeln. Im nicht genutzten Zustand werden die Austragöffnung und die Tropfenbildungsgeometrie vorzugsweise von einer auf das Gehäuse aufsteckbaren Schutzkappe geschützt.

Es wird primär eine besonders kompakte Bauweise vorgeschlagen, bei der der Tropfenspender ein flaches Gehäuse aufweist. Dieses flache Gehäuse beinhaltet den Flüssigkeitsspeicher und zumindest den wesentlichen Teil der Pumpeinrichtung. Als Gehäuse im Sinne der Erfindung werden alle strukturell wesentlichen Teile des Spenders angesehen, die im Betrieb zueinander ortsfest bleiben.

Unter einer flachen Bauweise wird eine Bauweise verstanden, bei der eine maximale Breite des Gehäuses die maximale Dicke des Gehäuses um mindestens 25% überschreitet, vorzugsweise um mindestens 50%, insbesondere vorzugweise um 100% oder mehr. Insbesondere bevorzugt ist es also, wenn das Gehäuse doppelt so breit wie dick ist. Die Länge des Gehäuses überschreitet vorzugsweise die Breite, insbesondere vorzugsweise um mindestens 50%.

Ein solches flaches Gehäuse ist bequem mitzunehmen und insbesondere geeignet, in einer Hosentasche verstaut zu werden. Vorzugsweise handelt es sich um einen vergleichsweise kleinen Spender mit einer Breite von vorzugsweise 3,6 cm oder mehr, jedoch insbesondere vorzugsweise weniger als 6 cm. Die Dicke des Gehäuses beträgt vorzuweise weniger als 1,8 cm, insbesondere vorzugsweise 1,5 cm oder weniger. Die Länge des Gehäuses beträgt vorzugsweise mindestens 4 cm, insbesondere vorzugsweise jedoch 5 cm oder mehr. Die Länge übersteigt vorzugsweise 10 cm nicht und beträgt insbesondere vorzugsweise 8 cm oder weniger.

Die Hauptachse eines erfindungsgemäßen Tropfenspenders erstreckt sich von einem Speicherboden zur Austragöffnung und/oder wird durch die Ausrichtung der Austragöffnung oder eine Mittelachse des Flüssigkeitsspeichers definiert.

Vorzugsweise ist ein Querschnitt des Gehäuses bezogen auf die Blickrichtung der Hauptachse zumindest in einem Teilbereich von vorzugsweise mindestens 40% der Länge des Gehäuses mit einem im Wesentlichen elliptischen Querschnitt versehen. Hierunter wird verstanden, dass der Querschnitt nicht rechteckig bzw. rechteckig mit viertelkreisförmig gerundeten Ecken gestaltet ist, sondern entsprechend einer Ellipse einen uneinheitlichen und im Bereich einer Vorderseite und einer Rückseite gegenüber den kurzen Seitenflächen größeren Krümmungsradius aufweist.

Diese elliptische Formgebung ist bei einem kompakten Spender erfindungsgemäßer Art von besonderem Vorteil, da die Rückseite mit entsprechend sanft gerundeter Formgebung sicher auf dem Zeigefinger, dem Mittelfinger und dem Ringfinger aufliegen kann, wenn eine gegenüberliegend auf der Vorderseite angeordnete Betätigungsfläche eingedrückt wird. Hierdurch wird begünstigt, den Tropfenspender gleichzeitig gut positionieren und dann sicher und gefühlvoll betätigen zu können.

Die kompakten Außenmaße des Gehäuses sind jedoch vorzugsweise mit einem dennoch vergleichsweise großen Flüssigkeitsspeicher kombiniert. Das Innenvolumen des Flüssigkeitsspeichers beträgt vorzugsweise mindestens5 ml, insbesondere vorzugweise mindestens 10 ml. Das Innenvolumen des Flüssigkeitsspeichers beträgt vorzugsweise nicht mehr als 30 ml.

Dabei beträgt ein Innenvolumen des Flüssigkeitsspeichers vorzugsweise mindestens 50 % des von einer Außenkontur des Tropfenspenders umschlossenen Volumens bei ggf. entfernter Schutzkappe, vorzugsweise mindestens 60 %. Es wird also vorzugsweise mindestens die Hälfte des Volumens des Tropfenspenders durch das Innenvolumen des Flüssigkeitsspeichers gebildet. Das von der Außenkontur des Tropfenspenders ohne Kappe umschlossene Volumen beträgt vorzugweise zwischen 10 cm³ und 60 cm³, insbesondere zwischen 15 cm³ und 30 cm³.

Dieser Aspekt der Erfindung betreffend das Verhältnis von Innenvolumen und Außenvolumen kann auch ohne die beschriebene flache Bauweise von Vorteil sein, wobei die weiteren in dieser Beschreibung des Spenders beschriebenen Unteraspekte auch bei einem solchen Spender ohne flache Bauweise zusätzlich vorgesehen sein können.

Um ein in Relation zum Volumen der Außenkontur großes Volumen des Flüssigkeitsspeichers zu erzielen, kann es von Vorteil sein, wenn der Flüssigkeitsspeicher sich in Richtung der Hauptachse bis in der Bereich der Pumpeinrichtung erstreckt, so dass sich bezogen auf die Hauptachse die Pumpeinrichtung, insbesondere deren Pumpkammer, auf gleicher Höhe wie der Flüssigkeitsspeicher befindet. Insbesondere kann der Flüssigkeitsspeicher bezogen auf die Breite des Gehäuses linksseitig und rechtsseitig der Pumpkammer jeweils einen Teilabschnitt des Innenvolumens des Flüssigkeitsspeichers aufweisen. Neben der Tatsache, dass eine überlappende Bauweise den verfügbaren Bauraum ideal ausnutzt, lässt sich durch die Überlappung der Pumpkammer mit dem Flüssigkeitsspeicher auch sehr einfach ein sehr breiter Einlass in die Pumpkammer erzielen, der begünstigt, dass sich hier keine Luft sammelt, die das Ansaugen von Flüssigkeit durch die Pumpeinrichtung stören könnte. Der Einlass zwischen dem Flüssigkeitsspeicher und der Pumpkammer beträgt vorzugsweise an der engsten Stelle nicht weniger als 2 mm².

Bei einem erfindungsgemäßen Tropfenspender erfolgt der Austrag mittels der bereits genannten Pumpeinrichtung. Diese ist innerhalb des Gehäuses vorgesehen und wird mittels einer eindrückbaren und dabei vorzugsweise schwenkbeweglichen Betätigungshandhabe betätigt. Sofern der Tropfenspender über eine Schutzkappe verfügt, so ist diese vorzugsweise derart angepasst, dass sie im aufgesetzten Zustand die Betätigungshandhabe überdeckt oder anderweitig eine ungewollte Betätigung der Betätigungshandhabe unterbindet.

Die Betätigungshandhabe kann an einer die Dicke des Spenders überspannenden Seitenfläche vorgesehen sein. Diese Bauweise kann bzgl. der Bauweise der Pumpeinrichtung von Vorteil sein, da die Pumpeinrichtung dann sehr einfach mit einem in der Querrichtung beweglichen Kolben ausgestaltet sein kann, wobei ein vergleichsweise großer Kolbenhub möglich ist.

Um eine kompakte Bauweise des Spenders und eine bequeme Betätigung zu begünstigen, ist es allerdings bevorzugt, wenn die Betätigungshandhabe auf einer die Breite überspannenden Vorderseite des Gehäuses vorgesehen ist, insbesondere in zentrischer Anordnung. Eine hier vorgesehene Betätigungshandhabe kann trotz der flachen Bauweise des Spenders vergleichsweise groß sein und damit eine angenehme und gut dosierte Betätigung gestatten. Die Betätigungshandhabe ist vorzugsweise in bezogen auf die Breite zentrischer Anordnung auf der Vorderseite angeordnet. Bezogen auf die Breite nimmt die Betätigungshandhabe vorzugsweise mehr als die Hälfte des verfügbaren Platzes auf der Vorderseite ein. Insbesondere vorzugsweise beträgt die Breite der Betätigungshandhabe mindestens 20 mm, insbesondere mindestens 25 mm. Insbesondere bei einer solchen vergleichsweise großen und an der Vorderseite angeordneten Betätigungshandhabe ist es von Vorteil, wenn die Schutzkappe die Betätigungshandhabe schützend überdecken kann und vorzugweise auch ausreichend steif ist, dass eine ungewollte Betätigung der Betätigungshandhabe bei aufgesetzter Schutzkappe unterbunden ist.

Die Betätigungshandhabe wirkt vorzugsweise auf eine Pumpeinrichtung mit einer orthogonal zur Hauptachse und zu Breite beweglichen Kolbeneinheit. Zwar ist aufgrund der dünnen Bauweise der Raum für eine solche Pumpeinrichtung und insbesondere ihren Pumpenhub hier sehr begrenzt. Die geringe Ausbringungsmenge je Betätigung von vorzugsweise zwischen 5 µl und 30 µl erlaubt jedoch trotz der flachen Bauweise die Anordnung einer geeigneten Pumpe. Bei der Kolbeneinheit kann es sich um eine Kolbeneinheit mit einem an einer Zylinderfläche gleitenden Kolben handeln. Aufgrund des geringen verfügbaren Bauraums ist jedoch eine Kolbeneinheit als Membranteil, das zumindest partiell aus einem elastisch verformbaren Material gefertigt ist, von Vorteil. Ein umlaufender Rand des Membranteils ist dabei an einem Pumpkammerbauteil angebracht, so dass insbesondere ein zentrischer Bereich unter der Einwirkung der Betätigungshandhabe zur gegenüberliegenden Rückseite hin gedrückt werden kann. Die Pumpeinrichtung verfügt vorzugsweise über ein Einlassventil und ein Auslassventil, insbesondere in Art druckabhängig öffnender Ventile. Die entsprechenden Kanäle, der Einlasskanal und der Auslasskanal, sind vorzugsweise auf der Innenseite der Rückseite des Gehäuses vorgesehen.

Die Kolbeneinheit weist vorzugsweise einen elastisch verformbaren Ausgleichsabschnitt auf, mittels dessen die Verbindung mit der Betätigungshandhabe gebildet ist, so dass die Schwenkbewegung der Betätigungshandhabe in eine translative Bewegung des Kolbens überführt werden kann.

Die Betätigungshandhabe selbst ist entweder rein translativ beweglich oder schwenkbeweglich zum Gehäuse. Vorzugsweise hebt sie sich farblich vom Gehäuse, insbesondere einer umgebenden Gehäuseschale, ab.

Die Betätigungshandhabe kann durch ein einzelnes Bauteil gebildet sein, welches über ein mehrteiliges Scharnier oder ein Filmscharnier mit einem Gehäuseteil verbunden ist. Auch ist eine Gestaltung mit mehrteiliger Betätigungshandhabe möglich. Hier kann ein Außenteil vorgesehen sein, welches insbesondere unter ästhetischen Gesichtspunkten gestaltet sein kann, sowie ein Innenteil, welches insbesondere der Kopplung mit einer Kolbeneinheit dienen kann und vor allem unter technischen Gesichtspunkten gestaltet ist. Ist die Betätigungshandhabe mehrteilig ausgestaltet, so kann insbesondere das Außenteil einstückig mit einem Gehäuseteil verbunden sein und/oder das Innenteil durch ein mehrteiliges Scharnier mit dem Gehäuse verbunden sein.

In Hinblick auf das Gehäuse selbst werden unterschiedliche Aspekte für besonders bevorzugenswert gehalten.

So ist bei einer bevorzugten Gestaltung vorgesehen, dass die Pumpeinrichtung eine Pumpkammer aufweist, die zumindest abschnittsweise durch ein Pumpkammerbauteil begrenzt ist. Dieses Pumpkammerbauteil, welches vorzugsweise auch einen Einlass vom Flüssigkeitsspeicher sowie einen Kanal zwischen Pumpkammer und Austragöffnung zumindest teilweise definiert, ist dabei einstückig ausgebildet mit einem Speicherwandungsbauteil, welches zumindest zum überwiegenden Teil eine begrenzende Wandung des Flüssigkeitsspeichers bildet. Dieses gemeinsame einstückige Bauteil stellt damit das Hauptbauteil des Spenders dar und gewährleistet insbesondere eine vollständige Abdichtung zwischen der Wandung und dem Pumpkammerbauteil. Weiterhin erleichtert eine solche einstückige Bauweise die ideale Nutzung des Innenraums des Gehäuses als Flüssigkeitsspeicher.

Dieser Aspekt des einstückigen Pumpkammer- und Speicherbauteils kann auch ohne die beschriebene flache Bauweise von Vorteil sein, wobei die weiteren in dieser Beschreibung des Spenders beschriebenen Unteraspekte auch bei einem solchen Spender ohne flache Bauweise zusätzlich vorgesehen sein können.

Vorzugsweise weist der Tropfenspender, insbesondere bei Gestaltung mit einem einstückigen Pumpkammer- und Speicherbauteil der beschriebenen Art, eine hiervon separate Gehäuseschale auf, die auf das Pumpkammerbauteil aufgeschoben ist, insbesondere von der Seite der Austragöffnung aus. Die Gehäuseschale kann insbesondere eine Durchbrechung für die Austragöffnung aufweisen, wobei es bevorzugt ist, wenn die Austragöffnung nicht unmittelbar durch die Gehäuseschale gebildet wird, sondern durch das durch die Durchbrechung hindurchragende Pumpkammer- und Speicherbauteil oder ein weiteres separates Gehäusebauteil. Hierdurch kann eine Gestaltung erreicht werden, bei der die separate Gehäuseschale bestimmungsgemäß nicht in Flüssigkeitskontakt gelangt, so dass bspw. bei der Materialwahl keine diesbezüglichen Bedingungen zu erfüllen sind.

Wenn die Betätigungshandhabe einstückig mit einem Gehäuseteil ausgebildet ist, so kann es sich insbesondere um die genannte Gehäuseschale handeln, an der die Betätigungshandhabe mittels Filmscharnier schwenkbar angelenkt ist. Alternativ kann die Betätigungshandhabe mittels eines mehrteiligen Scharniers schwenkbar gestaltet sein. Bei einem solchen Scharnier ist ein gehäuseseitiger Scharnierabschnitt vorzugsweise an einer Außenseite des Pumpkammerbauteils vorgesehen, insbesondere unterhalb der beschriebenen Gehäuseschale.

Ein Zwischenraum zwischen einer Außenseite des Pumpkammerbauteils und einer Innenseite der Gehäuseschale kann ergänzende Funktionen übernehmen. Insbesondere kann hier eine Rückstellfederzur Rückstellung der Betätigungshandhabe angeordnetsein, insbesondere in einer gegenüber der Pumpkammer versetzten Position. Weiterhin kann der Zwischenraum genutzt werden, um hier einen Belüftungskanal vorzusehen. Dabei kann es sich insbesondere um einen Kanal handeln, der zumindest in einem Teilabschnitt umfänglich vom Pumpkammerbauteil und der Gehäuseschale begrenzt wird. So kann eine Bauweise von Vorteil sein, bei der eine Nut an einer Außenseite des Pumpkammerbauteils vorgesehen ist, die zur Bildung eines Belüftungskanals, insbesondere eines Kapillarkanals, von der Innenseite der Gehäuseschale verschlossen wird, so dass sich ein Kanal bildet.

Insbesondere wenn ein einstückiges Pumpkammer- und Speicherbauteil vorgesehen ist, kann zusätzlich ein separater Speicherboden vorgesehen sein, der dicht mit dem Speicherwandungsbauteil verbunden ist. Der aufgrund der Formgebung des Gehäuses längliche Speicherboden schließt den Flüssigkeitsspeicher bodenseitig ab. Der Speicherboden kann einen Verschluss darstellen, der nach Befüllen des Flüssigkeitsspeichers am Speicherbauteil befestigt wird. Vorzugsweise wird jedoch der Speicherboden bereits vor der Befüllung mit dem Speicherbauteil fest verbunden, insbesondere durch Verschweißen oder Verkleben. Der Speicherboden kann statt des Pumpkammerbauteils für einen Belüftungskanal verwendet werden.

Neben der Bauweise mit einem einstückigen Pumpkammer- und Speicherbauteil kann auch eine Bauweise von Vorteil sein, bei der das Speicherwandungsbauteil und das Pumpkammerbauteil als separate Bauteile ausgebildet und miteinander umlaufend verbunden sind, wiederum vorzugsweise mittels einer Klebeverbindung oder eine Schweißverbindung. Ein separates Speicherbodenbauteil ist bei einer solchen Gestaltung nicht erforderlich. Der Boden kann daher einstückig mit der umgebenden Wandung das Speicherwandungsbauteil bilden.

Von Vorteil ist es, wenn das Speicherwandungsbauteil am dem Pumpkammerbauteil gegenüberliegenden Boden eine Befüllungsöffnung aufweist. Diese vorzugsweise runde Befüllungsöffnung verbleibt bei der Herstellung des Spenders offen und wird erst nach der Befüllung des Flüssigkeitsspeichers verschlossen, vorzugweise mittels eines Verschlusselements, welches mit dem Rand der Befüllungsöffnung verschnappt.

Das Speicherwandungsbauteil, welches den Flüssigkeitsspeicher zumindest teilweise umgibt, ist vorzugweise aus einem zumindest teiltransparenten Kunststoff-Material gefertigt. Insbesondere kann das Speicherwandungsbauteil einheitliche Materialeigenschaften aufweisen, so dass alle hierdurch gebildeten Wandungen lichtdurchlässig sind. Dabei sind sowohl glasklare als auch milchige Gestaltungen sowie eine weiße oder anderweitige Färbung möglich. Die Transparenz gestattet es, den Füllstand im Flüssigkeitsspeicher einfach zu erkennen. Die oben beschriebene Gehäuseschale kann oberhalb des Flüssigkeitsspeichers eine dort lichtundurchlässige Gehäusegestaltung ermöglichen.

Als Material für das Speicherbauteil oder das einstückige Pumpkammer- und Speicherbauteil kommen insbesondere Polypropylen (PP), Cycloolefin-Copolymer (COC), Cycloolefin-Polymer (COP) oder Polyethylenterephthalat (PET) in Frage. Vorzugsweise sind auch nahezu alle anderen Bauteile des Tropfenspenders und insbesondere alle Gehäuseteile aus diesen oder anderen Kunststoffen gefertigt. Wenn die Federn auch aus Kunststoff gefertigt sind, können auch alle Teile Kunststoffteile sein. Vorzugsweise sind die Kunststoffteile aus Kunststoffen, die in einem gemeinsamen Recycling-Prozess verarbeitet werden können.

Ein erfindungsgemäßer Spender weist vorzugsweise zusätzlich zu den Ventilen der Pumpeinrichtung ein der Austragöffnungvorgeschaltetes Auslassventil auf. Dieses verhindert das Eindringen von Keimen. Es ist vorzugsweise mittels einer Schließfeder in seine Schließrichtung gedrückt und öffnet bei ausreichend hohem Flüssigkeitsdruck. Vorzugsweise ist eine vergleichsweise große druckwirksame Ventilfläche von insbesondere mindestens 50 mm² vorgesehen, um eine vergleichsweise starke Schließfeder verwenden zu können und dennoch mit geringem Flüssigkeitsdruck das Ventil zur Flüssigkeitsabgabe öffnen zu können. Vorzugsweise ist das Auslassventil dafür ausgebildet, bei einem Flüssigkeitsdruck von weniger als 1000 mbar zu öffnen, insbesondere vorzugsweise bei einem Druck von weniger als 800 mbar oder einem Druck von weniger als 600 mbar.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 zeigt ein erstes Ausführungsbeispiel eines erfindungsgemäßen Tropfenspenders in einer Gesamtdarstellung.
Fig. 2A und 2B zeigen den Tropfenspender der Fig. 1 in zwei geschnittenen Darstellungen.
Fig. 3 verdeutlicht den Aufbau des Tropfenspenders der Fig. 1 aus einzelnen Gehäuseteilen.
Fig. 4A und 4B zeigen ein einstückiges Pumpkammer- und Speicherwandungsbauteil in einer ungeschnittenen und einer geschnittenen Darstellung.
Fig. 5A und 5B zeigen ein zweites Ausführungsbeispiel eines erfindungsgemäßen Tropfenspenders in zwei geschnittenen Darstellungen.
Fig. 6 und 7 zeigen ein drittes Ausführungsbeispiel eines erfindungsgemäßen Tropfenspenders mit abgenommener und aufgesetzter Schutzkappe.

### DETAILLIERTE BESCHREIBUNG DER AUSFÜHRUNGSBEISPIELE

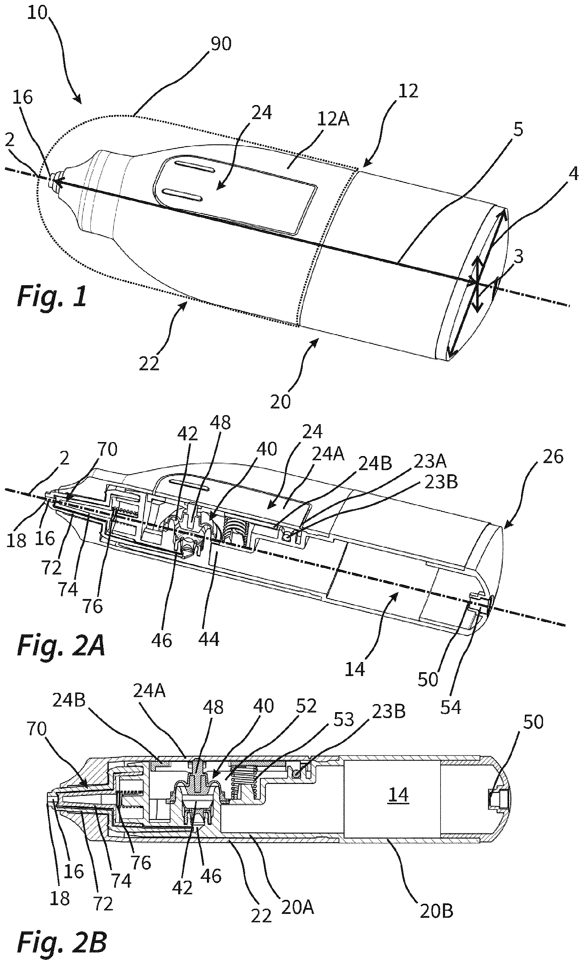

Die Fig. 1 bis 4B zeigen eine erste Ausgestaltung eines erfindungsgemäßen Tropfenspenders.

In Fig. 1 ist der Tropfenspender 10 in Gesamtdarstellung zu sehen, wobei eine Schutzkappe 90 durch gepunktete Darstellung angedeutet ist.

Der Tropfenspender 10 weist ein Gehäuse 12 auf. Dieses verfügt über eine Länge 5 in Richtung einer Hauptachse 2, die etwa 8 cm beträgt. Eine Breite 4 des Gehäuses 12 beträgt etwa 4 cm. Die Dicke 3 des Gehäuses beträgt 1,8 cm. Das Gehäuse 12 umgibt in Fig. 1 nicht entnehmbarer Weise einen Flüssigkeitsspeicher 14 sowie eine Pumpeinrichtung 40.

Zur Betätigung der Pumpeinrichtung 40 ist an einer Vorderseite 12A des Gehäuses 12 eine schwenkbewegliche Betätigungshandhabe 24 vorgesehen. Durch Eindrücken dieser Betätigungshandhabe 24 in einer Überkopfstellung kann Flüssigkeit aus dem Flüssigkeitsspeicher 14 zu einer Austragöffnung 16 gefördert werden. Diese ist von einer Tropfenbildungsgeometrie in Form eines Tropfenbildungsrings umgeben, so dass sich hier ausgetragene Flüssigkeit sammeln kann, bis die Menge der Flüssigkeit ausreicht, damit sich ein Tropfen des Tropfenspenders 10 von der Tropfenbildungsgeometrie 18 löst.

Das Gehäuse 12 weist über den größten Teil seiner Länge 5 hinweg einen elliptischen Querschnitt auf. Sowohl die Vorderseite 12A als auch die gegenüberliegende Rückseite sind also zur Seite hin sanft gewölbt ausgebildet. Das Gehäuse 12 liegt daher gut in der Hand des Benutzers, insbesondere auf Ringfinger, Mittelfinger und Zeigefinger, während mit dem Daumen die Betätigungshandhabe einfach, sicher und wohldosiert betätigt werden kann.

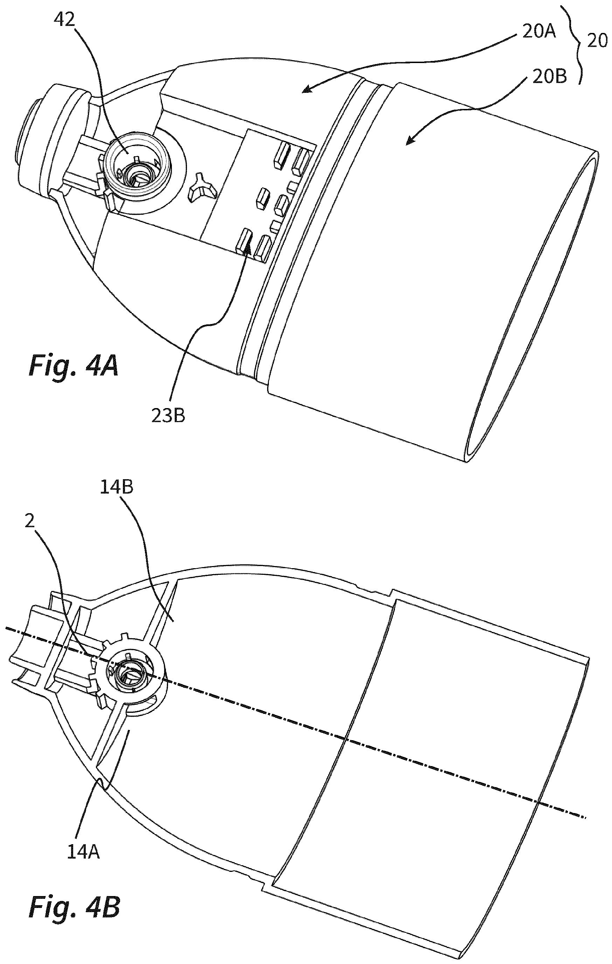

Die Fig. 2A und 2B zeigen den Tropfenspender 10 in geschnittener Ansicht. Hieraus ist zu ersehen, dass der Flüssigkeitsspeicher 14 einen Großteil des Innenraums des Gehäuses 12 einnimmt. Er ist, wie aus Fig. 4B zu ersehen ist, soweit in Richtung der Hauptachse 2 zur Austragöffnung 16 hin erstreckt, dass er partiell überlappend mit der Pumpeinrichtung 40 ausgebildet ist. Zwei Teilabschnitte 14A, 14B des Flüssigkeitsspeicher erstrecken sich bis auf die Höhe der Pumpkammer 42 der Pumpeinrichtung 40.

Anhand der Fig. 2A und 2B ist weiterhin gut zu erkennen, dass die Pumpeinrichtung 40 über eine orthogonal zur Vorderseite 12A verlagerbare Kolbeneinheit 48 verfügt, die als Membrankolbeneinheit ausgebildet ist. Dies bedeutet, dass die Kolbeneinheit 48 partiell aus einem elastisch verformbaren Material gefertigt ist und im Randbereich am Gehäuse 12 befestigt ist, vorliegend an einem im Weiteren noch näher erläuterten Pumpkammerbauteil 20A. Zusammen mit dem Pumpkammerbauteil 20A begrenzt die Kolbeneinheit 48 die genannte Pumpkammer42. Eingangsseitig und ausgangsseitig der Pumpkammer 42 sind im Bereich eines Einlasses 44 und eines Auslasses 46 Pumpenventile vorgesehen.

Wird die Betätigungshandhabe 24 gegen die Kraft einer Rückstellfeder 53 eingedrückt, so wird hierdurch die Kolbeneinheit 48 bezogen auf die Fig. 2B nach unten gedrückt und Flüssigkeit hierdurch in Richtung der Austragöffnung 16 gedrückt.

Der Austragöffnung 16 vorgeschaltet ist ein Auslassventil 70. Hierbei handelt es sich um ein Ventil, dessen primäre Funktion darin liegt, das Eindringen von Keimen zu vermeiden. Das Auslassventil 70 verfügt über eine zum Gehäuse 12 gehörige Auslassventiltülle 72, innerhalb dessen ein elastisch verformbarer Ventilkörper 74 angeordnet ist, der durch eine vergleichsweise starke Ventilfeder 76 in Richtung einer Schließstellung gedrückt wird. Der Ventilkörper 74 weist einen Druckbeaufschlagungskragen auf, der über eine Fläche von mehr als 100 mm² verfügt und daher bei anstehendem Flüssigkeitsdruck in der Lage ist, das Ventil gegen die Kraft der Ventilfeder 76 zu öffnen. Der Druck zum Öffnen des Auslassventils 70 liegt vorzugsweise unter 1000 mbar, insbesondere vorzugsweise unter 600 mbar.

Anhand der Fig. 2A und 2B ist weiterhin der Grundaufbau des Gehäuses 12 dieses ersten Ausführungsbeispiels zu erkennen. Das Gehäuse 12 weist beim Ausführungsbeispiel der Fig. 1 bis 4A ein gemeinsames Pumpkammer- und Speicherwandungsbauteil 20 auf. Dieses Bauteil umfasst zum einen das Pumpkammerbauteil 20A, welches zumindest partiell die Pumpkammer definiert und zusätzlich jenes Bauteil ist, gegen das sich die Rückstellfeder 53 der Betätigungshandhabe 24 sowie die Ventilfeder 76 des Auslassventils 70 stützt. Das Speicherwandungsbauteil 20B, welches einstückig mit dem Pumpkammerbauteil 20A ausgebildet ist, bildet einen insbesondere zylindrischen Wandungsring, der zum überwiegenden Teil den Flüssigkeitsspeicher 14 begrenzt. Bodenseitig ist ein Speicherbodenbauteil 26 vorgesehen, welches in das Speicherwandungsbauteil 20B eingeschoben ist und dort vorzugsweise verschweißt oder verklebt ist. Beim vorliegenden Ausführungsbeispiel ist im Speicherbodenbauteil 26 ein Belüftungskanal 50 vorgesehen, um Ausgleichsluft nach dem Austrag von Flüssigkeit aus dem Flüssigkeitsspeicher 14 in diesen einzubringen. Da die mit einem Tropfenspender 10 der beschriebenen Art ausgetragene Flüssigkeit vorzugsweise konservierungsmittelfrei ist, ist der Belüftungskanal 50 mit einem Sterilfilter 54 ausgebildet.

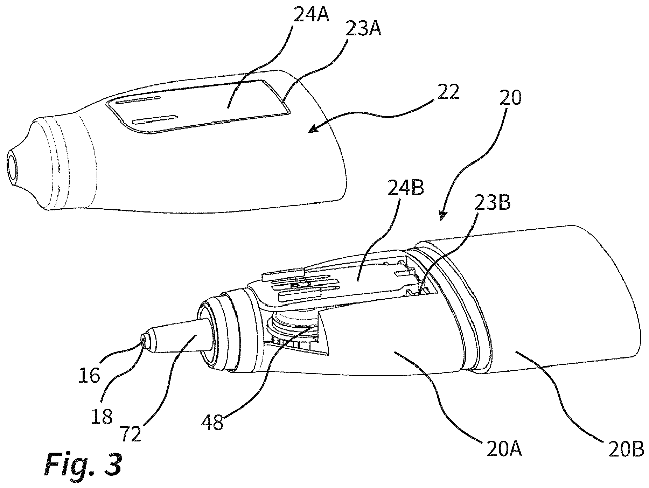

Anhand der Fig. 3 werden nochmals die hauptsächlichen Komponenten des Gehäuses 12 beschrieben. Das Gehäuse 12 verfügt über das bereits beschriebene Pumpkammer- und Speicherwandungsbauteil 20 mit seinen Teilabschnitten 20A, 20B. Ortsfest zu diesem Bauteil und fest mit dem Pumpkammerbauteil 20A verbunden ist die Auslassventiltülle 72 des Gehäuses 12. An der Außenseite des Pumpkammerbauteils 20A ist eine Vertiefung vorgesehen, in der die Kolbeneinheit 48 sowie ein Innenteil 24B der Betätigungshandhabe 24 angeordnet ist.

Zusätzlich weist das Gehäuse 12 die Gehäuseschale 22 auf, an der einstückig über ein Filmscharnier 23A ein Außenteil 24A der Betätigungshandhabe 24 vorgesehen ist. An einander gegenüberliegenden Enden weist die Gehäuseschale 22 eine Durchbrechung für die Austragöffnung bzw. die Auslassventiltülle 72 auf bzw. eine Öffnung, durch die das Pumpkammer- und Speicherwandungsbauteil 20 in die Gehäuseschale 22 eingeschoben werden kann. Vorzugsweise verrasten die Bauteile 20, 22 im gefügten Zustand.

Die Außenseite des Pumpkammerbauteils 20A und die Innenseite der Gehäuseschale 22 definieren im gefügten Zustand einen geschützten Zwischenraum 52, in dem die Rückstellfeder 53 angeordnet ist und die Kolbeneinheit48 umfänglich befestigt ist.

Die Fig. 4A und 4B zeigen in separater Darstellung das Pumpkammer- und Speicherwandungsbauteil 20. In Fig. 4A ist das Bauteil ungeschnitten gezeigt. Zu erkennen ist der zur Vorderseite offene Schacht, der nach Ergänzung der Kolbeneinheit 48 die Pumpkammer 42 bildet, ein Haltestift für die Rückstellfeder 53 sowie ein Teil des Scharniers 23B. Aus der geschnittenen Darstellung der Fig. 4B sind die Teilabschnitte 14A, 14B ersichtlich, die sich bezogen auf die Hauptachse 2 bis in den Bereich der Pumpkammer 42 erstrecken.

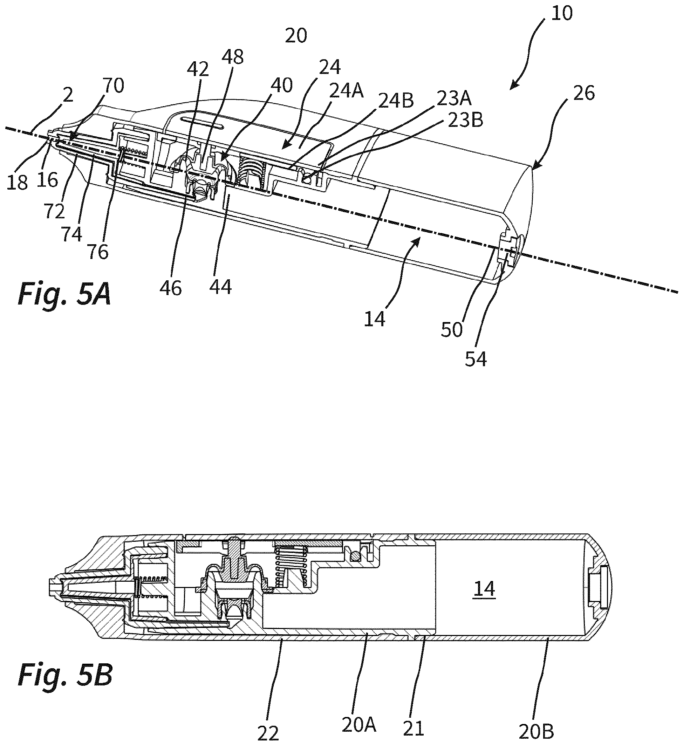

Fig. 5A und 5B zeigen eine alternative Bauweise. Hier ist das Speicherwandungsbauteil 20B nicht einstückig mit dem Pumpkammerbauteil 20A hergestellt, sondern erst nachträglich in einem Fügebereich 21 miteinander verbunden worden, insbesondere mittels einer Verschweißung oder Verklebung. Bei einer solchen Gestaltung kann dann auf ein separates Speicherbodenbauteil 26 verzichtet werden.

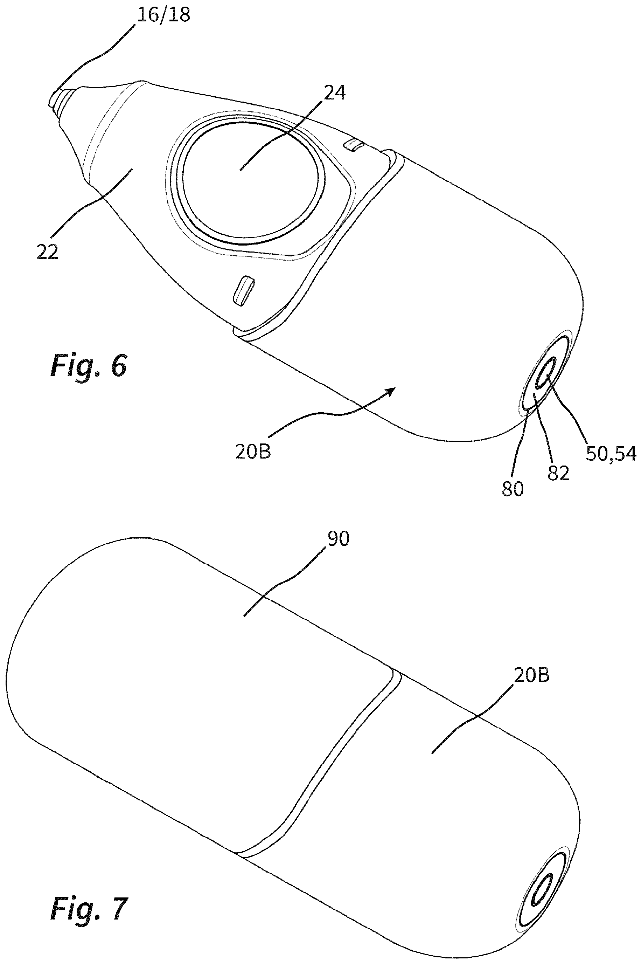

Die Fig. 6 und 7 zeigen ein drittes Ausführungsbeispiel eines Tropfenspenders 10. Dieser ist im Wesentlichen wie der Tropfenspender 10 der Fig. 5A und 5B aufgebaut. Ein Unterschied besteht in der allgemeinen Formgebung, insbesondere auch der Betätigungshandhabe 24. Zudem ist am Boden des Speicherwandungsbauteils 22B eine Befüllungsöffnung 80 vorgesehen, die durch Einschnappen eines Verschlusselements 82 nach der Befüllung verschlossen werden kann. Das Verschlusselement 82 kann einen Belüftungskanal50 und einen darin eingesetzten Sterilfilter 54 umfassen.

## Patentansprüche

1. Tropfenspender (10) zum tropfenweisen Austrag einer Flüssigkeit, insbesondere zum tropfenweisen Austrag einer pharmazeutischen Flüssigkeit, mit den folgenden Merkmalen:
a. der Tropfenspender (10) weist einen Flüssigkeitsspeicher (14) zur Lagerung der Flüssigkeit vor dem Austrag auf, und
b. der Tropfenspender (10) weist eine Austragöffnung (16) auf, der eine Tropfenbildungsgeometrie (18) zugeordnet ist, und
c. der Tropfenspender (10) weist eine Pumpeinrichtung (40) auf, mittels derer Flüssigkeit aus dem Flüssigkeitsspeicher (14) zur Austragöffnung (16) gefördert werden kann, so dass die Flüssigkeit sich an derTropfenbildungsgeometrie (18) sammelt und sich bei ausreichender gesammelter Flüssigkeitsmenge von der Tropfenbildungsgeometrie (18) löst,
**gekennzeichnet durch** die folgenden weiteren Merkmale:
d. der Tropfenspender (10) weist ein flaches Gehäuse (12) auf, innerhalb dessen der Flüssigkeitsspeicher und die Pumpeinrichtung angeordnet sind, wobei die Außenform des flachen Gehäuses (12) eine maximale Breite orthogonal zu einer Hauptachse (2) des Gehäuses aufweist, die mindestens 25% größer als die maximale Dicke der Außenform des Gehäuses (12) orthogonal zur Hauptachse und orthogonal zur Breite ist, und
e. zur Betätigung der Pumpeinrichtung (40) ist an einer Seitenfläche des Gehäuses (12) eine eindrückbare Betätigungshandhabe (24) vorgesehen.

2. Tropfenspender (10) nach Anspruch 1 mit dem folgenden weiteren Merkmal:
a. der Flüssigkeitsspeicher (14) erstreckt sich in Richtung der Hauptachse (2) überlappend bis in den Bereich der Pumpeinrichtung (40), insbesondere bis in den Bereich einer Pumpkammer (42) der Pumpeinrichtung (40),
vorzugweise mit dem folgenden zusätzlichen Merkmal:
b. der Flüssigkeitsspeicher (14) weist bezogen auf die Breite des Gehäuses linksseitig und rechtsseitig der Pumpkammer (42) jeweils einen Teilabschnitt (14A, 14B) eines Innenvolumens des Flüssigkeitsspeichers (14) auf.

3. Tropfenspender (10) nach Anspruch 1 oder 2 mit dem folgenden weiteren Merkmal:
a. ein Innenvolumen des Flüssigkeitsspeichers (14) beträgt mindestens 50 % des von einer Außenkontur des Tropfenspenders (10) umschlossenen Volumens, vorzugsweise mindestens 60 %.

4. Tropfenspender (10) nach einem der vorstehenden Ansprüche mit den folgenden weiteren Merkmalen:
a. die Pumpeinrichtung (40) weist eine Pumpkammer (42) auf, die zumindest abschnittsweise durch ein Pumpkammerbauteil (20A) begrenzt ist, und
b. der Flüssigkeitsspeicher (14) ist durch eine Wandung begrenzt, die zumindest zum überwiegenden Teil durch ein Speicherwandungsbauteil (20B) gebildet ist, und
c. es ist ein gemeinsames Bauteil (20) vorgesehen, welches das Pumpkammerbauteil (20A) und das Speicherwandungsbauteil (20B) einstückig bildet.

5. Tropfenspender (10) nach Anspruch 4 mit den folgenden weiteren Merkmalen:
a. der Tropfenspender (10) weist eine Gehäuseschale (22) auf, die auf das Pumpkammerbauteil (20A) aufgeschoben ist, und
b. die Gehäuseschale (22) weist eine Durchbrechung (22A) für die Austragöffnung (16) auf, vorzugweise mit den folgenden zusätzlichen Merkmalen:
c. die Betätigungshandhabe (24) ist einstückiger Teil der Gehäuseschale (22), und/oder
d. die Betätigungshandhabe (24) ist an der Gehäuseschale (22) oder an einer Außenseite des Pumpkammerbauteils (20A) gelagert, insbesondere schwenkbeweglich gelagert, und/oder
e. eine Außenseite des Pumpkammerbauteils (20A) und eine Innenseite der Gehäuseschale (22) definieren einen Zwischenraum (52), in dem vorzugsweise eine Rückstellfeder (60) zur Rückstellung der Betätigungshandhabe angeordnet ist,
f. der Tropfenspender weist einen Belüftungskanal auf, der zumindest abschnittsweise durch das Pumpkammerbauteil (20A) und die Gehäuseschale (22) gebildet wird, wobei vorzugsweise der Belüftungskanal zumindest in einem Teilabschnitt umfänglich vom Pumpkammerbauteil und der Gehäuseschale begrenzt wird.

6. Tropfenspender (10) nach einem der vorstehenden Ansprüche mit den folgenden weiteren Merkmalen:
a. der Tropfenspender (10) weist ein Speicherwandungsbauteil (20B) auf, welches den Flüssigkeitsspeicher (14) umfänglich umgibt, und
b. der Tropfenspender (10) weist einen Speicherboden (26) auf, der dicht mit dem Speicherwandungsbauteil (20B) verbunden ist,
vorzugsweise mit mindestens einem der folgenden weiteren Merkmale:
c. der Speicherboden (26) ist mittels einer Klebeverbindung oder einer Schweißverbindung mit dem Speicherwandungsbauteil (20B) verbunden, und/oder
d. der Speicherboden (26) ist von einem Belüftungskanal (50) durchdrungen.

7. Tropfenspender (10) nach einem der vorstehenden Ansprüche mit dem folgenden weiteren Merkmal:
a. der Tropfenspender (10) weist ein Speicherwandungsbauteil (20B) und ein Pumpkammerbauteil (20A) auf, die als separate Bauteile ausgebildet sind und miteinander umlaufend verbunden sind,
vorzugsweise mit mindestens einem der folgenden weiteren Merkmale:
b. das Pumpkammerbauteil (20A) ist mittels einer Klebeverbindung oder einer Schweißverbindung mit dem Speicherwandungsbauteil (20B) verbunden, und/oder
c. das Speicherwandungsbauteil (20B) weist am dem Pumpkammerbauteil (20A) gegenüberliegenden Boden eine Befüllungsöffnung auf, die vorzugsweise mittels eines Verschlusselements verschließbar ist.

8. Tropfenspender nach einem der vorstehenden Ansprüche mit den folgenden weiteren Merkmalen:
a. der Tropfenspender (10) weist ein Speicherwandungsbauteil (22B) auf, welches den Flüssigkeitsspeicher (14) zumindest teilweise umgibt, und
b. das Speicherwandungsbauteil (22B) ist aus einem zumindest teiltransparenten Material gefertigt,
vorzugsweise mit dem folgenden zusätzlichen Merkmal:
c. das Speicherwandungsbauteil weist als dominierenden Werkstoff Polypropylen (PP), Cycloolefin-Copolymer (COC), Cycloolefin-Polymer (COP) oder Polyethylenterephthalat (PET) auf.

9. Tropfenspender nach einem der vorstehenden Ansprüche mit dem folgenden weiteren Merkmal:
a. das Gehäuse (12) des Tropfenspenders (10) weist eine maximale Breite (4) von mindestens 2,5 cm und eine maximale Dicke (3) von höchstens 2 cm auf,
vorzugsweise mit mindestens einem der folgenden zusätzlichen Merkmale:
b. das Gehäuse (12) weist eine maximale Breite (4) von mindestens 3,6 cm auf, und/oder
c. das Gehäuse (12) weist eine maximale Dicke (3) von höchstens 1,8 cm auf, und/oder
d. das Gehäuse (12) weist eine Länge (5) zwischen 4 cm und 10 cm auf, vorzugsweise eine Länge (5) zwischen 5 cm und 8 cm, und/oder
e. die maximale Breite (4) des Gehäuses (12) ist mindestens 50% größer als die maximale Dicke des Gehäuses (12), insbesondere mindestens 100% größer.

10. Tropfenspender (10) nach einem der vorstehenden Ansprüche mit dem folgenden zusätzlichen Merkmal:
a. die Pumpeinrichtung (40) weist eine orthogonal zur Hauptachse (2) bewegliche Kolbeneinheit (48) auf,
vorzugsweise mit mindestens einem der folgenden zusätzlichen Merkmale:
b. die Kolbeneinheit (48) ist als Membranteil zumindest mit partiell aus einem elastisch verformbaren Material gefertigt und mit einem umlaufenden Rand an einem Pumpkammerbauteil (20A) befestigt, und/oder
c. die Kolbeneinheit (48) weist ein elastisch verformbaren Ausgleichsabschnitt auf, mittels dessen die Verbindung mit der Betätigungshandhabe (24) gebildet ist, und/oder
d. die Pumpeinrichtung (40) verfügt über einen Einlass (44) aus dem Flüssigkeitsspeicher (14), der an seiner schmalsten Stelle einen lichten Querschnitt von mindestens 2 mm² aufweist, gegebenenfalls auf eine Mehrzahl von zueinander parallel wirkenden Teilzuläufen.

11. Tropfenspender nach einem der vorstehenden Ansprüche mit einem der folgenden zusätzlichen Merkmale:
a. die Betätigungshandhabe (24) umfasst ein Außenteil (24A) und ein Innenteil (24B), und/oder
b. die Betätigungshandhabe (24) ist am Gehäuse (12) schwenkbeweglich angelenkt, und/oder
c. die Betätigungshandhabe (24) oder dessen Außenteil (24A) ist über ein Scharnier einstückig mit einer Gehäuseschale (22) des Gehäuses (12) verbunden, und/oder
d. die Betätigungshandhabe (24) oder dessen Innenteil (24B) ist mittels eines mehrteiligen Scharniers am Gehäuse (12) schwenkbar angelenkt, und/oder
e. die Betätigungshandhabe (24) oder zumindest ein Außenteil der Betätigungshandhabe weist eine von umgebenden Wandungen des Gehäuses abweichende Farbgebung auf, und/oder
f. die Betätigungshandhabe (24) weist eine Breite von mindestens 20 mm auf, insbesondere von mindestens 25 mm, und/oder die Betätigungshandhabe (24) weist eine Breite auf, die mindestens 50% der Breite des Gehäuses an seiner breitesten Stelle beträgt.

12. Tropfenspender (10) nach einem der vorstehenden Ansprüche mit dem folgenden zusätzlichen Merkmal:
a. der Querschnitt des Gehäuses (12) ist zumindest in einem Teilbereich mit einem im Wesentlichen elliptischen Querschnitt versehen,
vorzugweise mit dem folgenden weiteren Merkmal:
b. der Querschnitt des Gehäuses (12) ist zumindest über einen Teilbereich von mindestens 40% der Länge (5) des Gehäuses mit einem im Wesentlichen gleichbleibenden elliptischen Querschnitt versehen, wobei der Querschnitt des Gehäuses sich ausgehend von diesem Teilbereich zur Austragöffnung (16) hin verjüngt.

13. Tropfenspender nach einem dervorstehenden Ansprüche mit mindestens einem derfolgenden zusätzlichen Merkmale:
a. das Gehäuse (12) ist aus einem schwer verformbaren Kunststoff gefertigt, und/oder
b. der Flüssigkeitsspeicher (14) ist über einen Belüftungskanal (50) mit einer umgebenden Atmosphäre verbunden, wobei der Belüftungskanal (50) vorzugsweise mit einem Sterilfilter (54) und/oder einem Belüftungsventil versehen ist, und/oder
c. die Betätigungshandhabe (24) ist auf einer die Breite (4) überspannenden Vorderseite (12A) vorgesehen, vorzugsweise in zentrischer Anordnung,
d. der Tropfenspender (10) weist eine abnehmbare Schutzkappe (90) auf, wobei die Schutzkappe (90) vorzugsweise in einem aufgesetzten Zustand die Betätigungshandhabe (24) überdeckt, und/oder
e. der Tropfenspender (10) weist ein Auslassventil (70) auf, wobei das Auslassventil vorzugsweise dafür ausgebildet ist, bei einem Flüssigkeitsdruck von weniger als 1000 mbar zu öffnen, insbesondere vorzugsweise bei einem Druck von weniger als 800 mbar, insbesondere vorzuweise bei einem Druck von weniger als 600 mbar, und/oder
f. der Flüssigkeitsspeicher weist eine Befüllungsöffnung (80) auf, wobei die Befüllungsöffnung (80) vorzugsweise eine runde Form aufweist und vorzugsweise mittels eines Verschlusselements (82) verschließbar ist, insbesondere durch Verschnappen des Verschlusselements (82) an einem Rand der Befüllungsöffnung (80), und/oder
g. der Flüssigkeitsspeicher (14) ist mit einer pharmazeutischen Flüssigkeit befüllt, insbesondere einer konservierungsmittelfreien Flüssigkeit.

14. Tropfenspender (10) zum tropfenweisen Austrag einer Flüssigkeit, insbesondere zum tropfenweisen Austrag einer pharmazeutischen Flüssigkeit, mit den folgenden Merkmalen:
a. der Tropfenspender (10) weist einen Flüssigkeitsspeicher (14) zur Lagerung der Flüssigkeit vor dem Austrag auf, und
b. der Tropfenspender (10) weist eine Austragöffnung (16) auf, der eine Tropfenbildungsgeometrie (18) zugeordnet ist, und
c. der Tropfenspender (10) weist eine Pumpeinrichtung (40) auf, mittels derer Flüssigkeit aus dem Flüssigkeitsspeicher (14) zur Austragöffnung (16) gefördert werden kann, so dass die Flüssigkeitsich an derTropfenbildungsgeometrie (18) sammelt und sich bei ausreichender gesammelter Flüssigkeitsmenge von der Tropfenbildungsgeometrie (18) löst,
**gekennzeichnet durch** das folgende weitere Merkmal:
d. ein Innenvolumen des Flüssigkeitsspeichers (14) beträgt mindestens 50 % des von einer Außenkontur des Tropfenspenders (10) umschlossenen Volumens, vorzugsweise mindestens 60 %.

15. Tropfenspender (10) zum tropfenweisen Austrag einer Flüssigkeit, insbesondere zum tropfenweisen Austrag einer pharmazeutischen Flüssigkeit, mit den folgenden Merkmalen:
a. der Tropfenspender (10) weist einen Flüssigkeitsspeicher (14) zur Lagerung der Flüssigkeit vor dem Austrag auf, und
b. der Tropfenspender (10) weist eine Austragöffnung (16) auf, der eine Tropfenbildungsgeometrie (18) zugeordnet ist, und
c. der Tropfenspender (10) weist eine Pumpeinrichtung (40) auf, mittels derer Flüssigkeit aus dem Flüssigkeitsspeicher (14) zur Austragöffnung (16) gefördert werden kann, so dass die Flüssigkeit sich an derTropfenbildungsgeometrie (18) sammelt und sich bei ausreichender gesammelter Flüssigkeitsmenge von der Tropfenbildungsgeometrie (18) löst, **gekennzeichnet durch** die folgenden weiteren Merkmale:
d. die Pumpeinrichtung (40) weist eine Pumpkammer (42) auf, die zumindest abschnittsweise durch ein Pumpkammerbauteil (20A) begrenzt ist, und
e. der Flüssigkeitsspeicher (14) ist durch eine Wandung begrenzt, die zumindest zum überwiegenden Teil durch ein Speicherwandungsbauteil (20B) gebildet ist, und
f. es ist ein gemeinsames Bauteil (20) vorgesehen, welches das Pumpkammerbauteil (20A) und das Speicherwandungsbauteil (20B) einstückig bildet.
